# EUROPEAN PATENT APPLICATION

(11) **EP 2 975 033 A1**
(43) Date of publication of application: **20.01.2016**
(21) Application number: 14382283.1
(22) Date of filing: 17.07.2014
(51) Int. Cl.: C07D 295/195, C07C 271/22, A61K 31/27, A61K 31/5375, A61P 33/02, A61P 33/06, C07C 317/28

(54) **Improved cysteine-protease inhibitors**

(71) Applicant: Universitat Jaume I, 12071 Castelló de La Plana (ES)
(72) Inventor: González Adelantado, Florenci Vicent, 12071 Castelló de la Plana (ES); Rodríguez Pastor, Santiago, 12071 Castelló de la Plana (ES); Royo Calvo, Santiago, 12071 Castelló de la Plana (ES)
(74) Representative: Hoffmann Eitle

(57) **Abstract**

It is the object underlying the present invention to provide compounds, compositions and formulations that are useful for the treatment of parasitic diseases caused by a parasite species selected from the list consisting of the following genders: Leishmania, Trypanosome or Plasmodium, in particular selected from the following species: Leishmania donovani, Trypanosoma cruzi, Trypanosoma Brucei and Plasmodium falciparum.

## Description

### FIELD OF THE INVENTION

The present invention relates to new cysteine-protease inhibitors and pharmaceutical compositions containing them.

### BACKGROUND OF THE INVENTION

Trypanosomiasis (American and African trypanosomiasis), Leishmaniasis and malaria are major parasitic diseases in developing countries.

Chagas disease or American trypanosomiasis is a tropical disease caused by the protozoan parasite Trypanosoma cruzi which affects between 16 and 18 million people in Latin America causing 50.000 deaths every year. Twenty-five percent of Latin American population (90 million) is reckoned to be at risk of contracting the disease (Weekly Epidemiological Record, World Health Organization, 2000, 2:10-12).

African trypanosomiasis or sleeping sickness affects about half million people in sub-Saharan Africa, and 66 million people in 36 countries are reckoned to be at risk of contracting it. The eastern variant of the disease is caused by Trypanosoma brucei rhodesiense and the western variant by Trypanosoma brucei gambiense, the infectious agent being in both cases the tsetse fly, which is widely distributed throughout Africa. So far, no vaccine exists for American trypanosomiasis or Chagas disease. The drugs used (benznidazole and nifurtimox) are only useful in the early stages, show low efficacy and undesirable side effects. Furthermore, Melarsoprol is the only available drug for African trypanosomiasis or sleeping sickness. It consists of a toxic arsenic compound, which is administered with its antidote, and exposes 10% of individuals to risk of arsenic encephalopathy, from which almost 1.000 people die each year on account of drug toxicity. Recently, eflornithine has been developed for sleeping sickness treatment which, in spite of having proved to be effective and well tolerated, has shown many disadvantages.

Leishmaniasis is caused by a parasite called *Leishmania,* which is transmitted via the bites of a tiny phlebotomine sandfly infected with the parasite. Leishmaniasis occurs in 88 countries in South America, Africa, Middle East, Asia and southern Europe. Roughly 12 million people are affected with over 350 million at risk of infection. The disease is devastating in many ways; the cutaneous forms are debilitating and highly disfiguring, leading to social rejection, and the visceral forms are typically fatal. The current treatment of choice is amphotericin B, though dosing is limited by renal toxicities and there have also been suggestions of resistance. Miltefosine and paromomycin are emerging as effective therapies, however all available therapies have limitations including price, safety, effectiveness against all forms of the disease, and drug resistance.

Lastly, malaria is caused by protozoa of Plasmodium species, mainly Plasmodium falciparum and is transmitted to people by anopheles mosquitoes. Malaria affects nearly 40% of world's population where over three thousand million people are at risk, occurring in about one hundred countries from very depressed tropical areas of Africa, Asia and Latin America. Over 90% of cases and 80% of deaths occur in tropical Africa (World Malaria Report 2005). Although there are some drugs to prevent malaria such as chloroquine and mefloquine, these show side effects and none of them guarantees total protection.

All of these diseases (Trypanosomiasis, Leishmaniasis and malaria) have a similar infection cycle in which the infectious agent (insect) transmits the pathogen to man. All the pathogens of these diseases possess a common essential enzymatic activity for their life cycle, i.e., cysteine-protease activity: cruzain in Trypanosoma cruzi, rhodesain in T. Brucei and falcipain in Plasmodium falciparum (C. R. Caffrey et al, Mol. Biochem. Parasitol. 2001, vol. 1, pp. 61-73). Recent investigations directed to the search for drugs for these diseases is based on the inhibition of the above mentioned cysteine-proteases, so that the synthesis of inhibitors to these enzymes could give rise to new drugs for fighting diseases, specially as an alternative to traditional therapy in resistant organisms (J. H. McKerrow et al, Bioorg. Med. Chem. 1999, vol. 7, pp. 639-644).

To this effect, various peptide-based irreversible cysteine-protease inhibitors have been developed, for example, halomethyl ketones, diazomethanes, epoxysuccinyl derivatives, and vinyl sulfone derivatives (H. Otto et al, Chem. Rev. 1997, vol. 97, pp. 133-171). A disadvantage of chloromethyl ketones is their high reactivity (they react with serine-proteases and other molecules containing SH groups) and the consequent lack of selectivity thereby resulting in *in vivo* toxicity. In order to increase the selectivity and reduce the reactivity of these compounds, other less reactive molecules have been developed, for example monofluoromethyl ketones (D. Rasnick, Anal. Biochem. 1985, vol. 149, pp. 461-465) and epoxy derivatives (W. R. Roush et al, Bioorg. Med. Chem. Lett. 1998, pp. 2809-2812). However, these compounds are not effective *in vivo* due to their low bioavailability.

Thus, despite the research efforts spent in the past, treatment of such parasitic diseases is far from being satisfactory. Therefore, development of new compounds for the treatment of parasitic diseases such as Trypanosomiasis, Leishmaniasis and malaria is still of great interest.

### BRIEF DESCRIPTION OF THE INVENTION

It is the object underlying the present invention to provide compounds, compositions and formulations that are useful for the treatment of parasitic diseases caused by a parasite species selected from the list consisting of the following genders: Leishmania, Trypanosome or Plasmodium, in particular selected from the following species: Leishmania donovani, Trypanosoma cruzi, Trypanosoma Brucei and Plasmodium falciparum.

More particularly, it is the object underlying the present invention to provide compounds, compositions and formulations that are useful for the treatment of leishmaniasis, malaria, chagas disease or American trypanosomiasis and african trypanosomiasis or sleeping sickness.

The present invention thus provides compounds for use in a method for treatment of parasitic diseases caused by a parasite species of the gender Trypanosome, Leishmania or Plasmodium, as well as for use in therapy in general, wherein the compounds act as inhibitors of the enzyme activity of cysteine-proteases.

A group of suitable compounds which can be used according to the present invention includes compounds comprising the following formula: 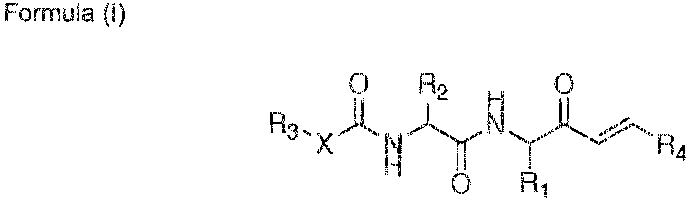 wherein
**R1** is -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3) -(C6H5), -CH(CH3)CH2(C6H5), -CH2CH(CH3)(C6H5), -CH2C6H4OH, -CH2CH2C6H4OH or -CH2CH(CH3)C6H4OH;
**R2** is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH or -CH2CH(CH3)C6H4OH;
**X** is -NH- or -O-
**R3** is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH, -CH2CH(CH3)C6H4OH and 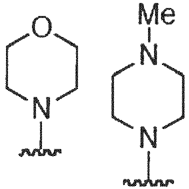
**R4** is -CO2R5, -COR5, -CONHR5 or -SO2R6; wherein **R5** is -CH3, -CH2CH3, -CH2CH2CH3, -CH2(C6H5) or -CH2CH2(C6H5) and **R6** is -CH3, -CH2CH3, -CH2CH2CH3 or 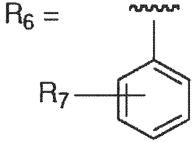 wherein **R7** is -H, -CH3, -CH2CH3, -CH2CH2CH3, -OCH3, -OCH2CH3, -OCH2CH2CH3, -NHCH3, -NHCH2CH3, -NHCH2CH2CH3, -SCH3, -SCH2CH3, -SCH2CH2CH3, -Cl, -Br or -I.

The present invention also provides methods of treatment using the inhibitors of the enzyme activity of cysteine-proteases described herein for the treatment of parasite diseases; a method for identifying an inhibitor; and a method of providing an inhibitor.

### DESCRIPTION OF THE INVENTION

The inventors have found new inhibitors of cysteine-proteases, in particular of any of the following cysteine-proteases cruzain, rhodesain, falcipain, cathepsin B or L.

The present invention thus provides compounds for use in therapy such as in a method for treatment of parasitic diseases caused by a parasite species selected from the list consisting of the following genders: Leishmania, Trypanosome or Plasmodium, in particular selected from the following species: Leishmania donovani, Trypanosoma cruzi, Trypanosoma Brucei and Plasmodium falciparum.

More particularly, the present invention provides compounds, compositions and formulations that are useful for the treatment of leishmaniasis, malaria, chagas disease or American trypanosomiasis and african trypanosomiasis or sleeping sickness.

Suitable compounds for use in the present invention comprise a moiety suitable as an alkylating site by reaction with the thiolate of the cysteine, an amino acid with a hydrophobic side chain that interacts with the hydrophobic pocket of the S2 site of the enzyme and an amino acid with a hydrophobic side chain that interacts with the S3 site. An example compound fulfilling all of these characteristics is illustrated in the scheme below:

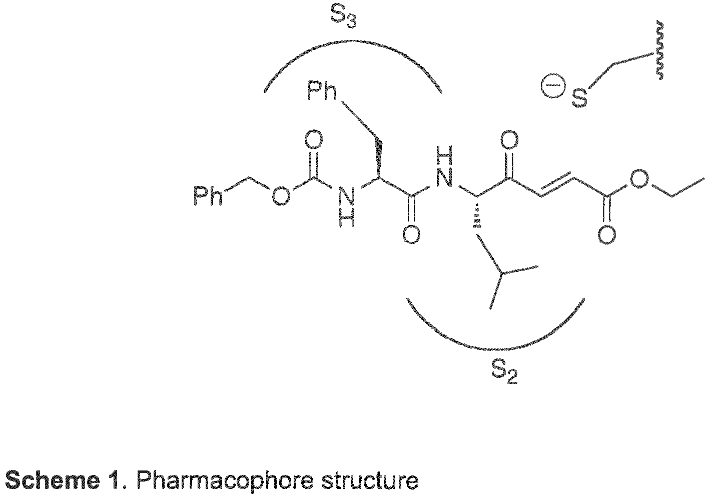

Therefore, suitable compounds for use in the present invention can be identified as follows.

First, the compounds to be used by the present invention must fulfil the following general pharmacophore structure: 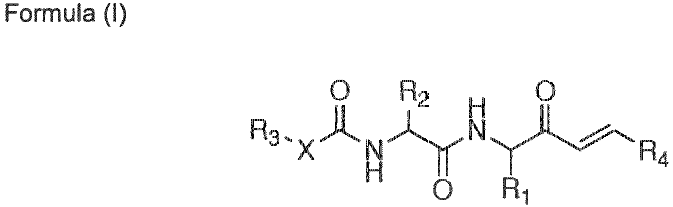 wherein
**R1** is -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3) -(C6H5), -CH(CH3)CH2(C6H5), -CH2CH(CH3)(C6H5), -CH2C6H4OH, -CH2CH2C6H4OH or -CH2CH(CH3)C6H4OH;
**R2** is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH or -CH2CH(CH3)C6H4OH;
**X** is -NH- or -O-
**R3** is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH, -CH2CH(CH3)C6H4OH and 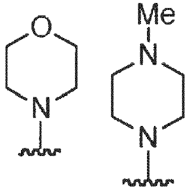
**R4** is -CO2R5, -COR5, -CONHR5 or -SO2R6; wherein **R5** is -CH3, -CH2CH3, -CH2CH2CH3, -CH2(C6H5) or -CH2CH2(C6H5) and **R6** is -CH3, -CH2CH3, -CH2CH2CH3 or 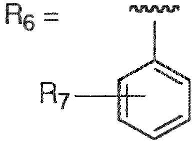 wherein **R7** is -H, -CH3, -CH2CH3, -CH2CH2CH3, -OCH3, -OCH2CH3, -OCH2CH2CH3, -NHCH3, -NHCH2CH3, -NHCH2CH2CH3, -SCH3, -SCH2CH3, -SCH2CH2CH3, -Cl, -Br or-I.

Secondly, the hits obtained fulfilling said pharmacophore structures are then subjected to a binding assay. It is expected that those compounds showing the above structure show an inhibitory action on all or any of the following cysteine-proteases: cruzain, rhodesain, falcipain, and cathepsin B or L.

In the context of the present invention, the term "inhibitory action" is understood as the ability of the inhibitor to block the cysteine protease activity. Preferably, the compounds have preferably an IC50 of the cysteine protease activity of from 50 µM to 1 pM, more preferably from 1 mM to 100 pM and still more preferably from 100 nM to 1 nM for inhibiting the cysteine-protease enzyme activity.

Therefore, compounds of the present invention are thus compounds capable of showing an inhibitory action on all or any of the following cysteine-proteases: cruzain, rhodesain, falcipain, and cathepsin B or L, and comprising the following formula: 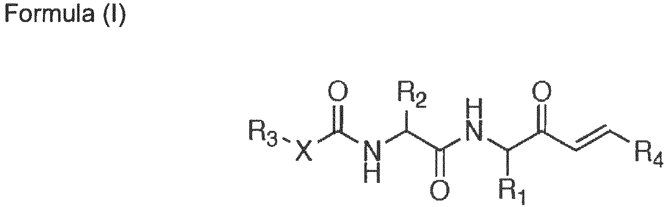 wherein
**R1** is -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3) -(C6H5), -CH(CH3)CH2(C6H5), -CH2CH(CH3)(C6H5), -CH2C6H4OH, -CH2CH2C6H4OH or -CH2CH(CH3)C6H4OH;
**R2** is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH or -CH2CH(CH3)C6H4OH;
**X** is -NH- or -O-
**R3** is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH, -CH2CH(CH3)C6H4OH and 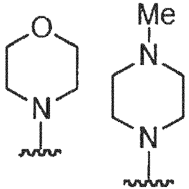
**R4** is -CO2R5, -COR5, -CONHR5 or -SO2R6; wherein **R5** is -CH3, -CH2CH3, -CH2CH2CH3, -CH2(C6H5) or -CH2CH2(C6H5) and **R6** is -CH3, -CH2CH3, -CH2CH2CH3 or 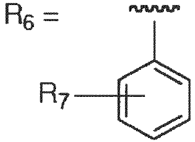 wherein **R7** is -H, -CH3, -CH2CH3, -CH2CH2CH3, -OCH3, -OCH2CH3, - OCH2CH2CH3, -NHCH3, -NHCH2CH3, -NHCH2CH2CH3, -SCH3, -SCH2CH3, -SCH2CH2CH3, -Cl, -Br or -I.

With regard to the meanings of R1, R2, R3, R4, X, R6, R5 and R7 it is particularly preferred to combine 2 of these meanings, even more preferred being the combination of 3, 4, 5, 6, 7, or 8 of these meanings.

Preferred compounds include the following:
Compound FGA-44 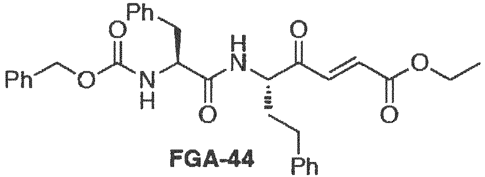
Compound FGA-47 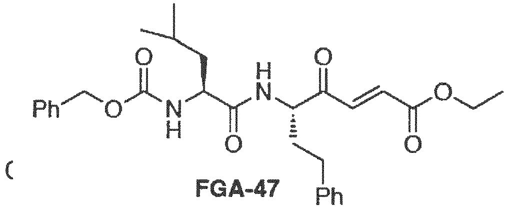 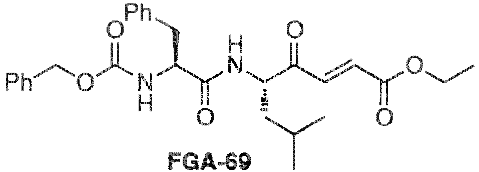
Compound FGA-70 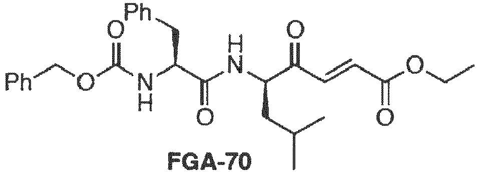
Compound FGA-74 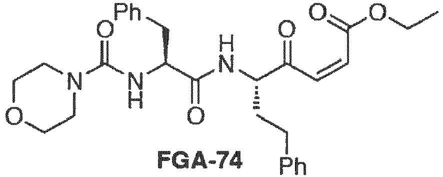
Compound FGA-75 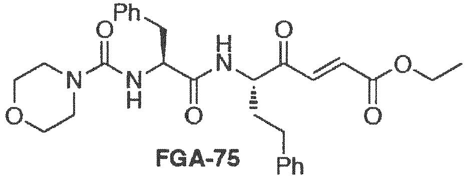
Compound FGA-76 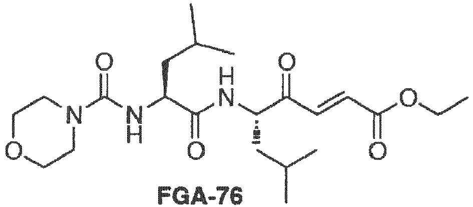
Compound FGA-77 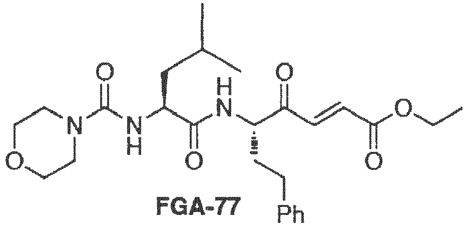
Compound FGA-72

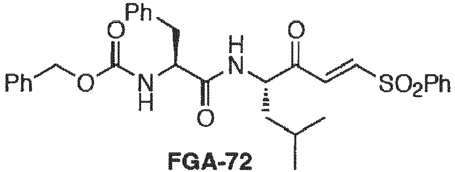

The compounds of Formula I can be prepared by the processes hereinafter described. It will be evident to a person skilled in the art that the precise method used for the preparation of a certain compound can vary depending on its chemical structure.

The general synthetic route for the preparation of compounds such as FGA-44, FGA-47, FGA-69, FGA-70, FGA-74, FGA-75, FGA-76 and FGA-77 is as follows:

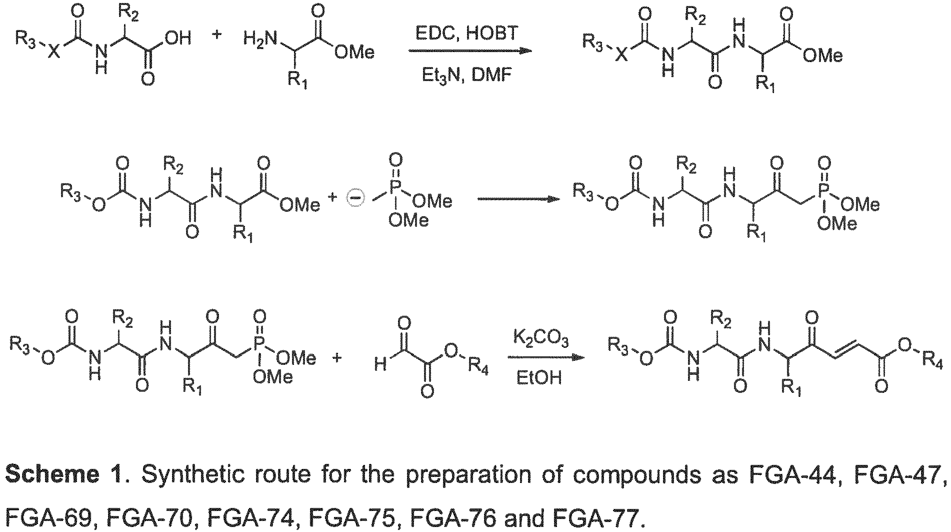

For merely illustrative purposes we herein incorporate the specific synthetic route used for the preparation of FGA-44.

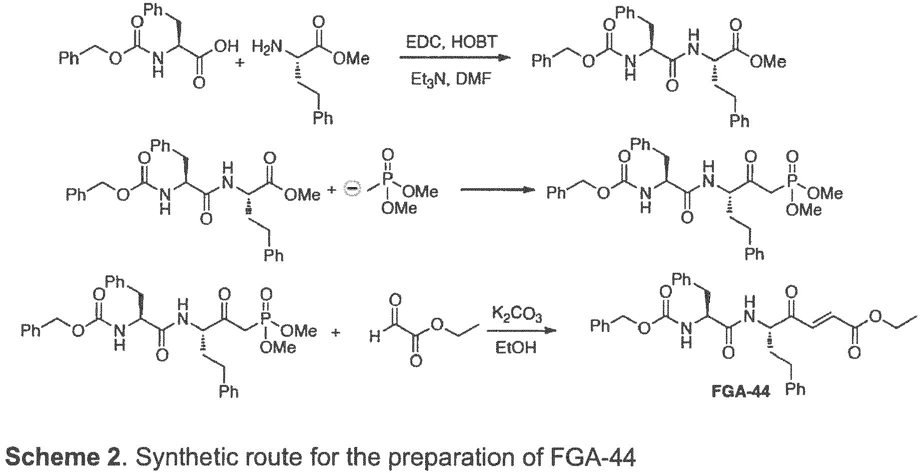

The general synthetic route for the preparation of compounds such as FGA-72 is as follows:

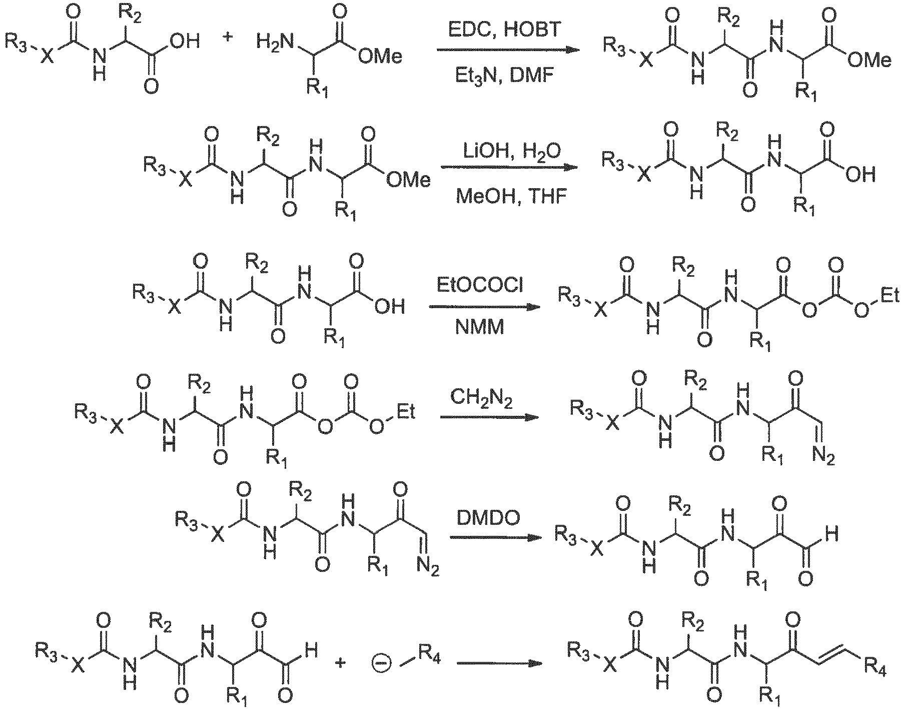

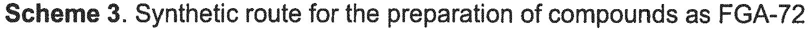

For merely illustrative purposes we herein incorporate the specific synthetic route used for the preparation of FGA-72.

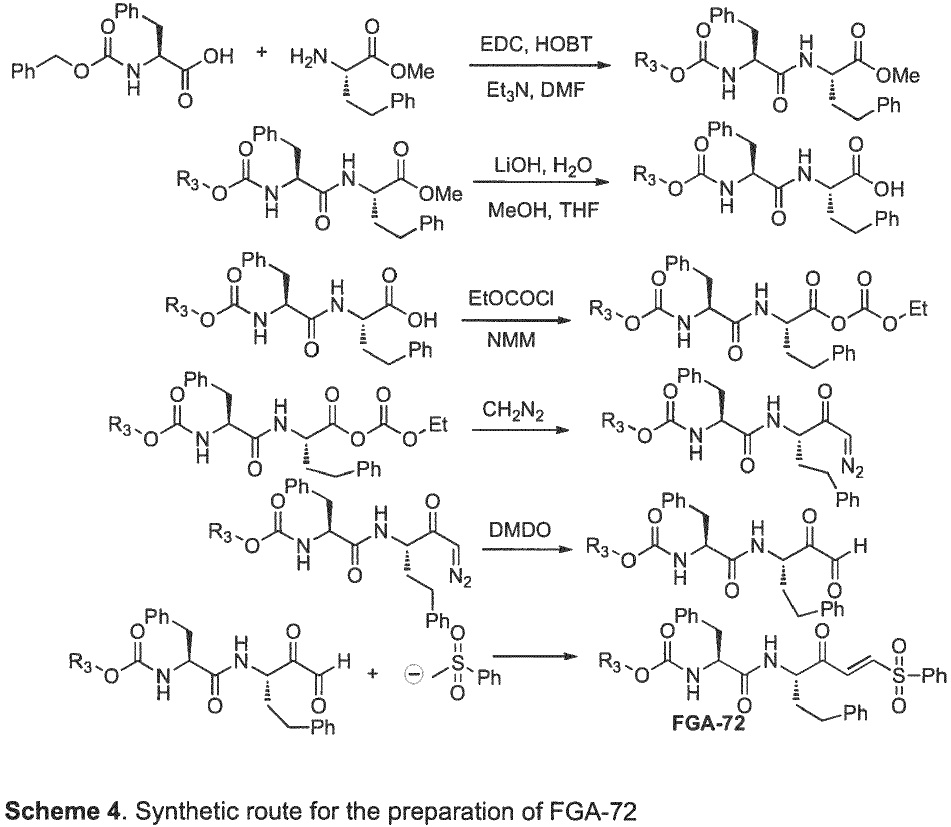

The compounds of the invention include pharmaceutically acceptable salts, esters, amides, and prodrugs therof, including but not limited to carboxylate salts, amino acid addition salts, esters, amides, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively non-toxic, inorganic and organic acid addition salts of compounds of the present invention. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobsonate, and laurylsulphonate salts, and the like. These may include cations based on the alkali and alkaline earth metals, such as sodium, lithium, potassium, calcium, magnesium, and the like, as well as non-toxic ammonium, quaternary ammonium, and amine cations including, but not limited to ammonium, tetramethylammonium, tetraethylammonium, methylamine, dimethylamine, trimethylamine, triethylamine, ethylamine, and the like, (See, for example, Berge S.M, et al, "Pharmaceutical Salts," J.Pharm.Sci., 1977;66:1-19 which is incorporated herein by reference).

However, it is noted that the carbonyl group indicated in general formula I below cannot be converted to a hydroxyl group.

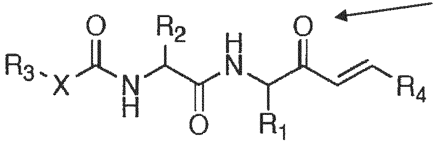

To demonstrate the above, the inventors have conducted enzymatic inhibition studies and IC50 determination on the following group of compounds in which the carbonyl group was replaced by a hydroxyl group.

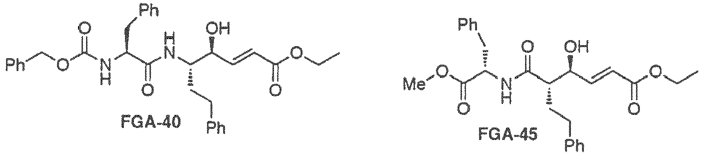

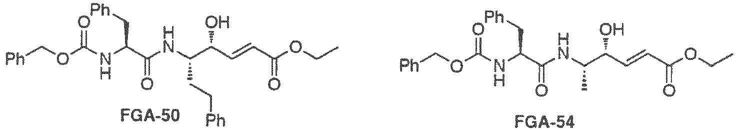

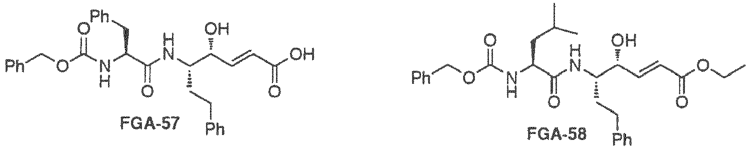

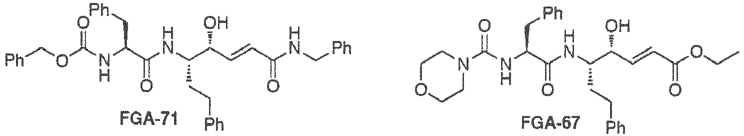

The results are illustrated in tables 1 and 2 below wherein CB=CathepsinB; CL=CathepsinL; FP2=Falcipain2; RD=Rhodesain and CR=Cruzain:

**Table 1. Enzymatic inhibition studies and IC50 determination of compounds of formula I having a hydroxyl group.**

| | CBIC50micM | CB (K2nd(M -1s-1) | CL(IC50) | CL(K2nd(M -1s-1) | FP 2(IC50) | FP 2(K2nd(M -1s-1) | RD (IC50) | RD(K2nd(M -1s-1) | CR(IC50) | CR(K2nd(M -1s-1) |
|---|---|---|---|---|---|---|---|---|---|---|
| 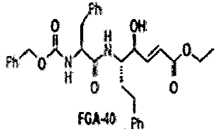 | 53 | 14 | 47 | 77 | - | - | - | - | - | - |
| 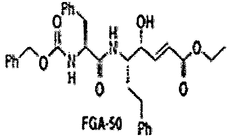 | 24 | 772 | 13 | 1127 | 34,4 | 557 | 9,5 | 3207 | - | - |
| 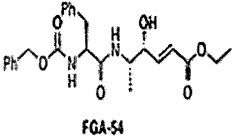 | - | - | 14,3 | 828 | 26,8 | 2,735 | 45,06 | 10951 | - | - |
| 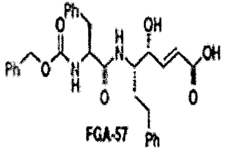 | - | - | 20 | 882 | 75 | 29 | - | - | - | - |
| 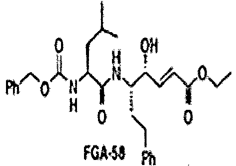 | - | - | - | - | - | - | - | - | - | - |
| 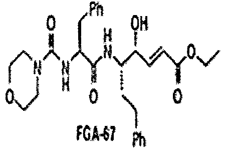 | 1,13 | 4190 | 2,45 | 1860 | | | 1,13 | 36735 | 3,75 | 3615 |
| 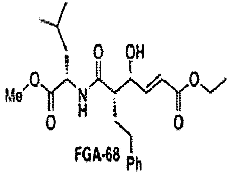 | | | | | | | | | | |
| 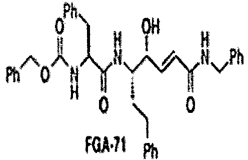 | - | - | - | - | - | - | - | - | - | - |

**Table 2. Enzymatic inhibition studies and IC50 determination of compounds of formula I.**

| | CBIC50micM | CB(K2nd(M-1s-1) | CL(IC50) | CL(K2nd(M-1s-1) | FP2(IC50) | FP2(K2nd(M-1s-1) | RD(IC50) | RD(K2nd(M-1s-1) | CR(IC50) | CR(K2nd(M-1s-1) |
|---|---|---|---|---|---|---|---|---|---|---|
| 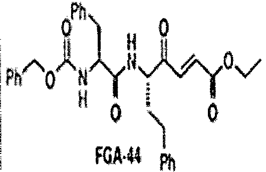 | 0,07 | 45486 | 0,05 | 45364 | 0,05975 | 17099 | 0,0307 | 1274510 | | |
| 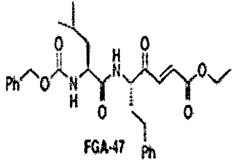 | 0,05 | 42393 | 0,2567 | 29885 | 0,5761 | 6035 | 0,1446 | 116147 | 0,077 | 60784 |
| 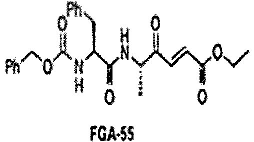 | 0,14 | 4738 | 0,09 | 197273 | 0,19 | 28285 | 0,59 | 6481 | 0,29 | 139583 |
| 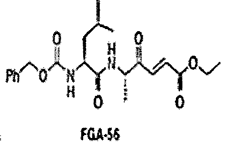 | 0,07 | 85470 | 0,55 | 9821 | 1,18 | 12013 | 0,8 | 14207 | 0,63 | 14412 |
| 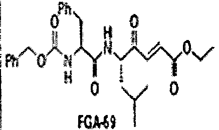 | 0,053 | 138462 | 0,0096 | 758621 | | | 0,01635 | 1611111 | 0,0162 | 516667 |
| 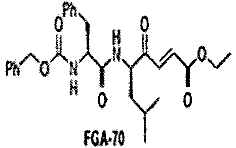 | 0,0642 | 116667 | 0,00815 | 704478 | | | 0,02595 | 1533333 | 0,0182 | 311828 |
| 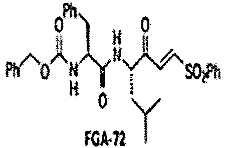 | 0,05 | 82248 | 0,00995 | 383272 | | | 0,006 | 1361750 | 0,0111 | 709411 |

As illustrated in tables 1 and 2 above, compounds of formula I in which the carbonyl group was replaced by a hydroxyl group resulted to be weak inhibitors of cysteine proteases. In contrast, compounds of formula I maintaining the carbonyl group resulted to be very potent inhibitors of assayed cysteine proteases with IC₅₀ values in the nanomolar-picomolar range. The k_{2nd} values are in the order of magnitude of 10⁵-10⁶ M⁻¹·s⁻¹ which denote these compounds to be potent irreversible inhibitors.

The present invention also provides the use of the compounds of formula I for therapy, such as for the treatment of malaria, leishmaniasis, chagas disease or American trypanosomiasis and african trypanosomiasis or sleeping sickness.

Accordingly, a further aspect of the present invention includes pharmaceutical compositions comprising one or more compounds of the invention disclosed above, associated with a pharmaceutically acceptable carrier. For administration, the compounds are ordinarily combined with one or more adjuvants appropriate for the indicated route of administration. The compounds may be admixed with lactose, sucrose, starch powder, cellulose esters of alkanoic acids, stearic acid, talc, magnesium stearate, magnesium oxide, sodium and calcium salts of phosphoric and sulfuric acids, acacia, gelatin, sodium alginate, polyvinylpyrrolidine, and/or polyvinyl alcohol, and tableted or encapsulated for conventional administration. Alternatively, the compounds of this invention may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

The term "prodrug" refers to compounds that are rapidly transformed in vivo to yield the parent compound of the above formulae, for example, by hydrolysis in blood. A thorough discussion of prodrugs is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are hereby incorporated, by reference.

The compounds disclosed in the present invention can be administered individually or in combination, usually in the form of a pharmaceutical composition. Such compositions are prepared in a manner well known in the pharmaceutical art and comprise at least one active compound.

The compounds and compositions of the present invention are useful for the treatment, prevention or amelioration of one or more symptoms of parasitic diseases caused by a parasite species selected from the list consisting of the following genders: leishmania, trypanosome or plasmodium, in particular selected from the following species: Leishmania donovani, Trypanosoma cruzi, Trypanosoma Brucei and Plasmodium falciparum.

Particularly, the compounds under Markush formula I are useful for the treatment, prevention or amelioration of one or more symptoms of malaria, leishmaniasis, chagas disease or American trypanosomiasis and african trypanosomiasis or sleeping sickness.

The present invention also provides a method for identifying compounds that can be used according to the present invention. Hence, the present invention provides a method for screening a compound from a library of compounds wherein said compound must comprise the pharmacophore structure of formula I and wherein said compound is subjected to a binding assay for determining whether the compound shows an inhibitory action on all or any of the following cysteine-proteases: cruzain, rhodesain, falcipain, and cathepsin B or L, and selecting those compound(s) that show said inhibitory action. The present invention also provides a method for providing a compound that comprises the pharmacophore structure of formula I, subjecting said compound to a binding assay for determining whether the compound shows an inhibitory action on all or any of the following cysteine-proteases: cruzain, rhodesain, falcipain, and cathepsin B or L, and selecting those compound(s) that show said inhibitory action, and synthesizing the compound or ordering the compound from a provider.

Throughout the description and claims the word "comprise" and variations of the word are not intended to exclude other technical features, additives, components, or steps.

Additional objects, advantages and features of the invention will become apparent to those skilled in the art partly upon examination of the description and partly by the practice of the invention.

The following examples are provided by way of illustration, and they are not intended to be limiting of the present invention.

### EXAMPLES

All the solvents used in the reactions were distilled at the time of being used by means of suitable drying agents. 1H-NMR and 13C-NMR spectra were recorded in COCI3 solution (1H, 7.24 ppm; 13C 77.0 ppm) at 30° C. on a 300 MHz Mercury Varian or 500 MHz Innova Varian RMN spectrometer at the "Serveis Centrals d'Instrumentació Cientifica de la Universitat Jaume I". Mass spectra were recorded on a QTOF I mass spectrometer (quadrupole-hexapole-TOF) with orthogonal Z-spray-electrospray interface (Micromass, Manchester, UK). IR spectra were recorded on oily films on NaCl tablets in a Perkin-Elmer 2000 FT-IR spectrometer. For column chromatography, EM Science Silica Gel 60 was used, whereas TLC was performed on E. Merck silica gel coated aluminum foils (Kieselgel 60, F254, 0.25 mm). Except when indicated otherwise, all the reactions were carried out under nitrogen atmosphere with magnetic stirring.

### Example 1. Route of synthesis of the compositions of the invention.

### 1.1. Route of synthesis of formula I compounds wherein R4 is not SO2R6.

The general synthetic route for the preparation of compounds such as FGA-44, FGA-47, FGA-69, FGA-70, FGA-74, FGA-75, FGA-76 and FGA-77 is the following:

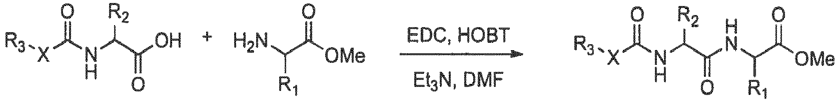

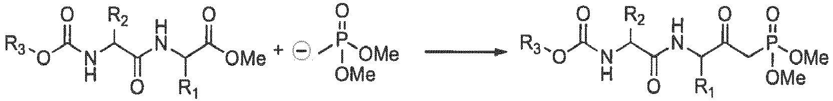

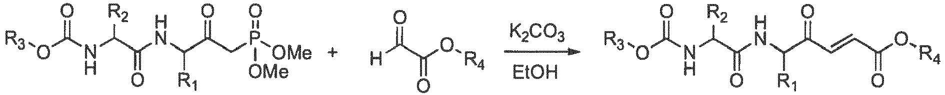

### 1.2. Route of synthesis of formula I compounds wherein R4 is SO2R6.

The general synthetic route for the preparation of compounds such as FGA-72 is the following: 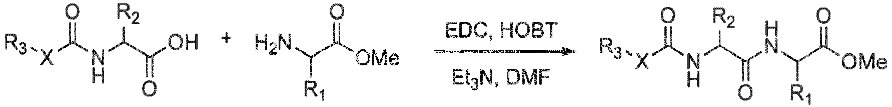 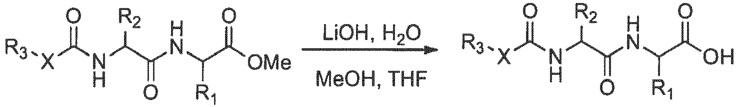 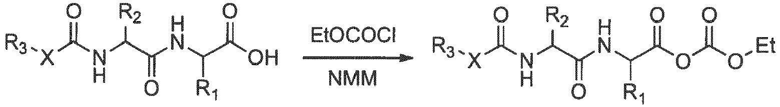 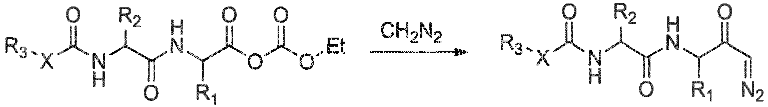 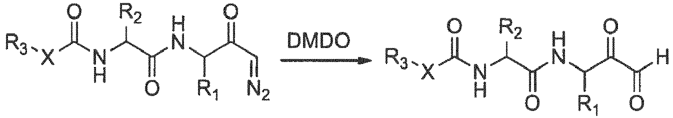 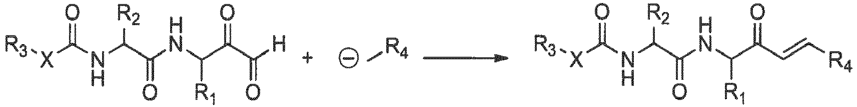 2.

### Example 2. Enzymatic inhibition studies and IC50 determination

### 2.1. Materials and methods

### 2.1.1. Protease Assays as shown in tables 1 and 2: Cathepsin L (human), Cathepsin B (human), Rhodesain (Trypanosoma brucei rhodesiense), Falcipain 2 (Plasmodium falciparum)

Enzyme assays used to identify inhibitors of the target cysteine proteases. The effect is detected measuring fluorescence increase due to hydrolysis of fluorogenic substrates by the target enzymes in absence and presence of potential inhibitors.

### MATERIALS

a. *Proteins (enzymes):*
   - Cathepsin L (human liver) Calbiochem
   - Cathepsin B (human liver) Calbiochem
   - Rhodesain: recombinantly expressed according to ref.: Caffrey, C. R.; Hansell, E.; Lucas, K. D.; Brinen, L. S.; Hernandez, A. A.; Cheng, J.; Gwaltney, S. L., II; Roush, W. R.; Stierhof, Y. D.; Bogyo, M.; Steverding, D.; McKerrow, J. H. Active site mapping, biochemical properties and subcellular localization of rhodesain, the major cysteine protease of Trypanosoma
   - Falcipain 2: recombinantly expressed according to ref.: Pandey, K. C.; Wang, S. X.; Sijwali, P. S.; Lau, A. L.; McKerrow, J. H.; Rosenthal, P. J. The Plasmodium falciparum cysteine protease falcipain-2 captures its substrate, hemoglobin, via a unique motif. Proc. Natl. Acad. Sci. U.S.A. 2005, 102, 9138-9143.
b. *Buffers:*
   - Cathepsin B: 50 mM Tris-Puffer pH 6.5 mit 2 mM DTT, 5 mM EDTA, 200 mM NaCl, 0.005% Brij35
   - Cathepsin L: 50 mM Tris-Puffer pH 6.5 mit 2 mM DTT, 5 mM EDTA, 200 mM NaCl, 0.005% Brij35
   - Rhodesain: 50 mM Acetat-Puffer pH 5.5 mit 5 mM DTT, 5 mM EDTA, 200 mM NaCl, 0.005% Brij35
   - Falcipain 2: 50 mM Acetat-Puffer pH 5.5 mit 5 mM DTT, 5 mM EDTA, 200 mM NaCl, 0.005% Brij35
c. *Substrates:*
   - Cathepsin L: 6,25 µM Z-Phe-Arg-7-amino-4-methylcumarin, HCl von Bachem, Ex 380 nm, Em 460 nm
   - Cathepsin B: 100 µM Z-Phe-Arg-7-amino-4-methylcumarin, HCl von Bachem, Ex 380 nm, Em 460 nm
   - Rhodesain: 10µM Z-Phe-Arg-7-amino-4-methylcumarin, HCl von Bachem, Ex 380 nm, Em 460 nm
   - Falcipain 2: 25µM Z-Phe-Arg-7-amino-4-methylcumarin, HCl von Bachem, Ex 380 nm, Em 460 nm
   - *Reference substance(s)*/*positive control(s):* E64
d. *Solvent for inhibitors and substrates:* DMSO 99,5% (GC) DC4540, Sigma
e. *Measurement device:* Tecan Fluorescence spectrophotometer infinite F200 Pro
f. *Well plates:* 96 well plates, Lia-Plate White, Greiner bio-one, flat bottom, high binding
g. Methodology: Substrates and inhibitor stock solutions are prepared in DMSO and diluted with the respective assay buffer (final DMSO concentration: 10%). Enzyme stock solutions are diluted with assay buffer to yield a fluorescence increase of dF/min ≈ _20. Total volume: 200 µL (180 µL of assay buffer, 5 µL of enzyme solution, 10 µL of DMSO or inhibitor solution, 5 µL of substrate solution). Assays are started by the addition of substrate solution. Fluorescence increase is detected over a period of 10 - 30 min, depending on time dependent (30 min) or non-time dependent (10 min) inhibition.
h. Temp: 25 °C
i. DATA-ANALYSIS
   - Software: Grafit 5.013
   - Ki values are calculated from IC50 values (obtained by the 4-parameter IC50 equation) using the Chen-Prusoff equation: Ki=IC50/(1 + [S]/Km)

### 2.2. Results of the enzymatic inhibition studies

In vitro inhibition of cysteine-proteases cruzain (CR) (Trypanosoma cruzi (T. cruzi)), rhodesain (RD) (T. brucei), cathepsin B (CB), falcipain (FP2) (Plasmodium falciparum) and cathepsin L (CL) was assessed by quantification of the resulting fluorescence emission of the proteolytic breakdown of synthetic compound Z-Phe-Arg-AMC (Bachem). The results of this determination are shown in tables 1 and 2 illustrated above.

### Example 3. Biological assays against Protozoa by using FGA-47

For all the susceptibility assays with parasites and cells, each drug was tested in duplicate and each assay was repeated at least once.
a. *Activity against Trypanosoma brucei rhodesiense STIB900.* This stock was isolated in 1982 from a human patient in Tanzania and after several mouse passages cloned and adapted to axenic culture conditions (Baltz et al 1985) Minimum Essential Medium (50 µl) supplemented with 25 mM HEPES, 1g/l additional glucose, 1% MEM non-essential amino acids (100x), 0.2 mM 2-mercaptoethanol, 1 mM Na-pyruvate and 15% heat inactivated horse serum was added to each well of a 96-well microtiter plate. Serial drug dilutions of eleven 3-fold dilution steps covering a range from 100 to 0.002 µg/ml were prepared. Then 4x10³ bloodstream forms of *T. b. rhodesiense* STIB 900 in 50 µl was added to each well and the plate incubated at 37 °C under a 5 % CO₂ atmosphere for 70 h. 10 µl Alamar Blue (resazurin, 12.5 mg in 100 ml double-distilled water) was then added to each well and incubation continued for a further 2-4 h (Raz et al 1997). Then the plates were read with a Spectramax Gemini XS microplate fluorometer (Molecular Devices Cooperation, Sunnyvale, CA, USA) using an excitation wave length of 536 nm and an emission wave length of 588 nm. The IC50 values were calculated by linear regression (Huber 1993) from the sigmoidal dose inhibition curves using SoftmaxPro software (Molecular Devices Cooperation, Sunnyvale, CA, USA). Melarsoprol (Arsobal Sanofi-Aventis, received from WHO) is used as control.
*b. Activity against T. cruzi.* Rat skeletal myoblasts (L-6 cells) were seeded in 96-well microtitre plates at 2000 cells/well in 100 µL RPMI 1640 medium with 10% FBS and 2 mM I-glutamine. After 24 h the medium was removed and replaced by 100 µl per well containing 5000 trypomastigote forms of *T. cruzi* Tulahuen strain C2C4 containing the β-galactosidase (Lac Z) gene (Buckner et al. 1996). After 48 h the medium was removed from the wells and replaced by 100 µl fresh medium with or without a serial drug dilution of eleven 3-fold dilution steps covering a range from 100 to 0.002 µg/ml. After 96 h of incubation the plates were inspected under an inverted microscope to assure growth of the controls and sterility. Then the substrate CPRG/Nonidet (50 µl) was added to all wells. A color reaction developed within 2-6 h and could be read photometrically at 540 nm. Data were analyzed with the graphic programme Softmax Pro (Molecular Devices), which calculated IC₅₀ values by linear regression (Huber 1993) from the sigmoidal dose inhibition curves. Benznidazole is used as control (IC50 0.5±0.2mg/ml).
c. *Activity against L. donovani axenic amastigotes.* Amastigotes of L. *donovani* strain MHOM/ET/67/L82 were grown in axenic culture at 37 °C in SM medium (Cunnigham et al. 1977) at pH 5.4 supplemented with 10% heat-inactivated fetal bovine serum under an atmosphere of 5% CO₂ in air. One hundred microlitres of culture medium with 10⁵ amastigotes from axenic culture with or without a serial drug dilution were seeded in 96-well microtitre plates. Serial drug dilutions of eleven 3-fold dilution steps covering a range from 90 to 0.002 µg/ml were prepared. After 70 h of incubation the plates were inspected under an inverted microscope to assure growth of the controls and sterile conditions. 10 µl of Alamar Blue (12.5 mg resazurin dissolved in 100 ml distilled water) (Mikus and Steverdig. 2000) were then added to each well and the plates incubated for another 2 h. Then the plates were read with a Spectramax Gemini XS microplate fluorometer (Molecular Devices Cooperation, Sunnyvale, CA, USA) using an excitation wave length of 536 nm and an emission wave length of 588 nm. Data were analyzed using the software Softmax Pro (Molecular Devices Cooperation, Sunnyvale, CA, USA). Decrease of fluorescence (= inhibition) was expressed as percentage of the fluorescence of control cultures and plotted against the drug concentrations. From the sigmoidal inhibition curves the IC₅₀ values were calculated.
*d. Activity againt Leishmania donovani intracellular amastigotes: macrophage assay:* Mouse peritoneal macrophages (4 x 10⁴ in 100 µl RPMI 1640 medium with 10% heat-inactivated FBS) were seeded into wells of Lab-tek 16-chamber slides. After 24 hrs 1.2 x 10⁵ amastigote *Leishmania donovani* in 100 µl were added. The amastigotes were taken from an axenic amastigote culture grown at pH 5.4. Four hrs later the medium containing free amastigote forms was removed and replaced by fresh medium. Next day the medium was replaced by medium containing different compound dilutions. Parasite growth in the presence of the drug was compared to control wells. After 96 hours of incubation the medium was removed and the slides fixed with methanol for 10 min followed by a staining with a 10% Giemsa solution. Infected and non-infected macrophages were counted for the control cultures and the ones exposed to the serial drug dilutions. The infection rates were determined. The results were expressed as % reduction in parasite burden compared to control wells, and the IC₅₀ calculated by linear regression analysis. Yang M, Arai C, Bakar Md A, Lu J, Ge JF, Pudhom K, Takasu K, Kasai K, Kaiser M, Brun R, Yardley V, Itoh I, Ihara M. 2010. Fluorinated rhodacyanine (SJL-01) possessing high efficacy for visceral leishmaniasis (VL). J Med Chem 14;53(1):368-73.
e. *Activity against P. falciparum.* In vitro activity against erythrocytic stages of *P*. *falciparum* was determined using a ³H-hypoxanthine incorporation assay (Desjardins et al. 1979, Matiel and Pink. 1990), using the drug sensitive NF54 strain (Schipol Airport, The Netherlands, Ponnudurai et al. 1981) and the standard drug chloroquine (Sigma C6628). Compounds were dissolved in DMSO at 10 mg/ml and added to parasite cultures incubated in RPMI 1640 medium without hypoxanthine, supplemented with HEPES (5.94 g/l), NaHCO₃ (2.1 g/l), neomycin (100 U/ml), Albumax^{R} (5 g/l) and washed human red cells A⁺ at 2.5% haematocrit (0.3% parasitaemia). Serial drug dilutions of eleven 3-fold dilution steps covering a range from 100 to 0.002 µg/ml were prepared. The 96-well plates were incubated in a humidified atmosphere at 37 °C; 4% CO₂, 3% O₂, 93% N₂. After 48 h 50 µl of ³H-hypoxanthine (=0.5 µCi) was added to each well of the plate. The plates were incubated for a further 24 h under the same conditions. The plates were then harvested with a Betaplate™ cell harvester (Wallac, Zurich, Switzerland), and the red blood cells transferred onto a glass fibre filter then washed with distilled water. The dried filters were inserted into a plastic foil with 10 ml of scintillation fluid, and counted in a Betaplate™ liquid scintillation counter (Wallac, Zurich, Switzerland). IC₅₀ values were calculated from sigmoidal inhibition curves by linear regression (Huber 1993) using Microsoft Excel. Chloroquine and artemisinin are used as control.

### 3.1. IC50 (micrograms/mL)determination by using FGA-47

| | | **T.b.rhod. IC50** | | | **T.cruzi. IC50** | | | **L.don. ax am IC50** | | | **P.falc NF54 IC50** | | |
|---|---|---|---|---|---|---|---|---|---|---|---|---|---|
| ID No | STI No. | repl1 | repl2 | **mean** | repl1 | repl2 | **mean** | repl1 | repl2 | **mean** | repl1 | repl2 | **mean** |
| Melarsoprol | | 0,005 | 0,002 | **0,003b** | | | | | | | | | |
| Benznidazole | | | | | 0,657 | 0,522 | **0,59** | | | | | | |
| Miltefosine | | | | | | | | 0,046 | 0,071 | **0,059** | | | |
| Chloroquine | | | | | | | | | | | 0,003 | 0,002 | **0,003** |
| FGA-47 | 397,14 | 1,68 | 0,675 | **1,1775** | 5,17 | 2,09 | **3,63** | 0,044 | 0,052 | **0,048** | 0,921 | 0,72 | **0,821** |

### 3.2. IC50 (micrograms/mL)determination by using FGA-44

| | | **T.b.rhod. IC50** | | | **T**.**cruzi**. **IC50** | | | **L**.**don**. **ax am IC50** | | | **P.falc NF54 IC50** | | |
|---|---|---|---|---|---|---|---|---|---|---|---|---|---|
| ID No | STI No. | repl1 | repl2 | **mean** | repl1 | repl2 | **mean** | repl1 | repl2 | **mean** | repl1 | repl2 | **mean** |
| Melarsoprol | | 0,003 | 0,004 | **0,004** | | | | | | | | | |
| Benznidazole | | | | | 0,367 | 0,664 | **0,516** | | | | | | |
| Miltefosine | | | | | | | | 1,44 | | **1,44** | | | |
| Chloroquine | | | | | | | | | | | 0,002 | 0,002 | **0,002** |
| FGA-44 | 446,13 | 0,446 | 0,564 | **0,505** | 7,74 | 14,1 | **10,92** | 6,87 | | **6,87** | 7,19 | 6,44 | **6,815** |

## Claims

1. A compound comprising the following formula: 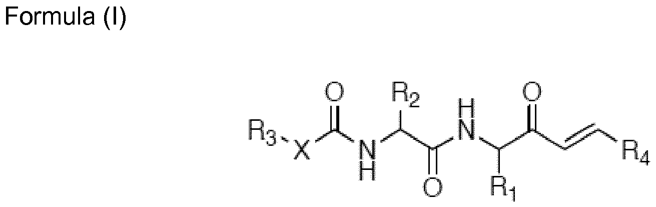 wherein
R1 is -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3) -(C6H5), -CH(CH3)CH2(C6H5), -CH2CH(CH3)(C6H5), -CH2C6H4OH, -CH2CH2C6H4OH or -CH2CH(CH3)C6H4OH;
R2 is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH or -CH2CH(CH3)C6H4OH;
**X** is -N H- or -O-
**R3** is -CH3, -CH2CH3, -CH2CH2CH3, -CH(CH3)2, -CH(CH3)CH2CH3, -CH2CH(CH3)2, -Phenyl, -CH2(C6H5), -CH2CH2(C6H5), -CH2CH2CH2(C6H5), -CH(CH3)(C6H5), -CH(CH3)CH2(C6H5), -CH2CH(CH3)(C6H5), -CH2C6H4OH, -CH2CH2C6H4OH, -CH2CH(CH3)C6H4OH and 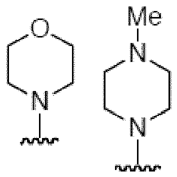
**R4** is -CO2R5, -COR5, -CONHR5 or -SO2R6; wherein **R5** is -CH3, -CH2CH3, -CH2CH2CH3, -CH2(C6H5) or -CH2CH2(C6H5) and **R6** is -CH3, -CH2CH3, -CH2CH2CH3 or 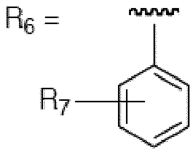
wherein **R7** is -H, -CH3, -CH2CH3, -CH2CH2CH3, -OCH3, -OCH2CH3, -OCH2CH2CH3, -NHCH3, -NHCH2CH3, -NHCH2CH2CH3, -SCH3, -SCH2CH3, -SCH2CH2CH3, -CI, -Br or -I.

2. The compound of claim 1, wherein said compound is selected from the list consisting of:
a. 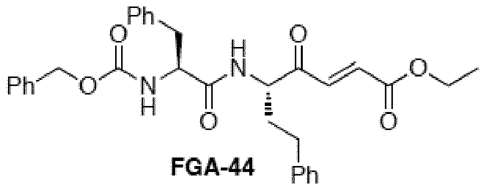
b. 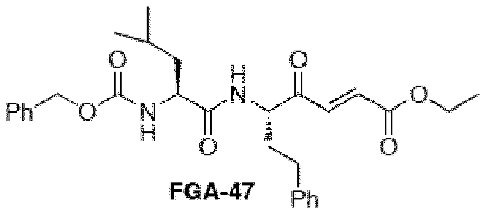
c. 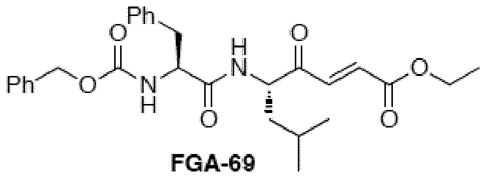
d. 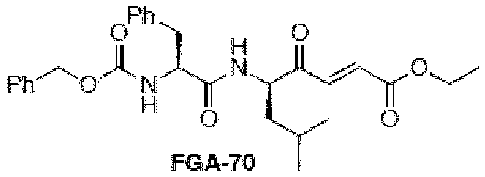
e. 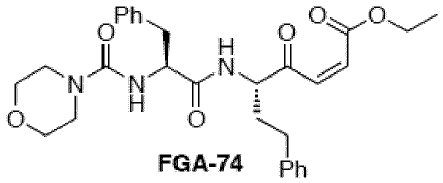
f. 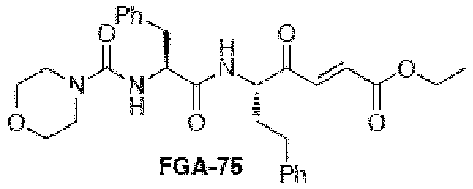
g. 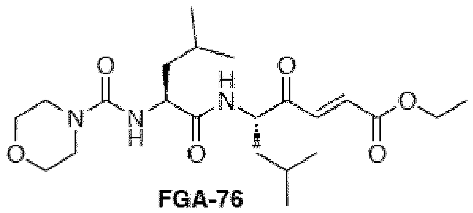
h. 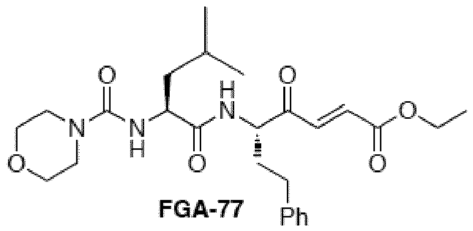 and
i. 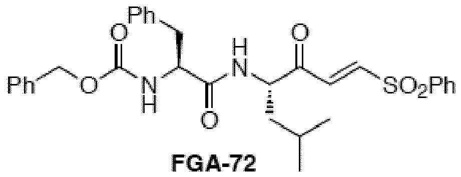
or a salt, ester, amide, and prodrug thereof.

3. The compound of any of claims 1 or 2, for use as a medicament.

4. A compound for use in a method for treatment of parasitic diseases caused by a parasite species selected from the list consisting of the following genders: trypanosome or plasmodium, wherein the compound fulfils the structure shown in formula I and is capable of showing an inhibitory action on all or any of the following cysteine-proteases: cruzain, rhodesain, falcipain, and cathepsin B or L.

5. The compound for use in a method for treatment according to claim 4, wherein the compound has an IC50 of the cysteine protease activity of from 50 µM to 1 pM for inhibiting the enzyme activity of any of the following cysteine-proteases: cruzain, rhodesain, falcipain, and cathepsin B or L.

6. The compound for use according to claim 4, wherein said compound is selected from the list of compounds as defined in claim 2.

7. The compound for use in a method for treatment according to any of claims 4 to 6, wherein the parasite species are selected from the list consisting of: Trypanosoma cruzi, Trypanosoma Brucei, leishmania and Plasmodium falciparum.

8. The compound for use in a method for treatment according to anyone of claims 4 to 7, wherein the parasitic diseases are selected from the list consisting of malaria, chagas disease or American trypanosomiasis and african trypanosomiasis or sleeping sickness.

9. A method for screening a compound from a library of compounds comprising the following steps:
a) Selecting a compound comprising the pharmacophore structure of formula I
b) Subjecting the compound selected in step a) to a binding assay for determining whether the compound shows an inhibitory action on all or any of the following cysteine-proteases: cruzain, rhodesain, falcipain, and cathepsin B or L, and
c) selecting those compound(s) that show said inhibitory action.

10. A method for providing a compound that comprises the following steps:
a) Selecting a compound comprising the pharmacophore structure of formula I;
b) Subjecting the compound selected in step a) to a binding assay for determining whether the compound shows an inhibitory action on all or any of the following cysteine-proteases: cruzain, rhodesain, falcipain, and cathepsin B or L, and
c) selecting those compound(s) that show said inhibitory action; and
d) synthesizing the compound or ordering the compound from a provider.
